Europäisches Patentamt

European Patent Office

Office européen des brevets

(19)

(11) Veröffentlichungsnummer: **0 184 055**

**A1**

(12) # EUROPÄISCHE PATENTANMELDUNG

(21) Anmeldenummer: 85114559.9

(22) Anmeldetag: 16.11.85

(51) Int. Cl.⁴: **C 07 D 307/32**
**//B01J23/86**

(30) Priorität: 04.12.84 DE 3444107
23.03.85 DE 3510569

(43) Veröffentlichungstag der Anmeldung:
11.06.86 Patentblatt 86/24

(84) Benannte Vertragsstaaten:
AT BE CH DE FR GB IT LI NL SE

(71) Anmelder: HÜLS AKTIENGESELLSCHAFT
- RSP Patente / PB 15 - Postfach 13 20
D-4370 Marl 1(DE)

(72) Erfinder: Nehring, Rudolf, Dr.
Griesheimer Strasse 12
D-4370 Marl(DE)

(72) Erfinder: Otte, Werner, Dr.
Eichenstück 5
D-4270 Dorsten 11(DE)

(54) Verfahren zur Herstellung von gamma -Butyrolacton durch katalytische Hydrierung von Maleinsäure-, Fumarsäure- und/oder Bernsteinsäureester.

(57) Zur Herstellung von ?-Butyrolacton hydriert man Maleinsäure-, Fumarsäure- oder Bernsteinsäureester bzw. deren Gemische in Gegenwart von Kupferchromit-Katalysatoren, vorzugsweise Barium-enthaltenden Kupferchromit-Katalysatoren in der Rieselphase bei einem Wasserstoffdruck von 1 bis 12 bar und Temperaturen von 180 bis 260 °C.

EP 0 184 055 A1

CHEMISCHE WERKE HÜLS AG          - 1 -          O.Z. 4030/4061
-PATENTABTEILUNG-

Verfahren zur Herstellung von γ-Butyrolacton durch katalytische Hydrierung von Maleinsäure-, Fumarsäure- und/oder Bernsteinsäureester"

Die Erfindung betrifft ein Verfahren zur selektiven Herstellung von γ-Butyrolacton aus leicht zugänglichen Estern, die sich von Maleinsäureanhydrid bzw. der Maleinsäure, der Fumarsäure und/oder der Bernsteinsäure ableiten, in Gegenwart von üblichen Kupferchromit-Katalysatoren. γ-Butyrolacton ist eine bekannte, im Handel befindliche Chemikalie, die als Zwischenprodukt zur Synthese z. B. von Pyrrolidon, N-Methyl-Pyrrolidon, N-Vinyl-Pyrrolidon und Methionin verwendet wird.

Nach bekannten Verfahren erhält man γ-Butyrolacton beispielsweise durch Hydrierung von Maleinsäure bzw. Maleinsäureanhydrid, die beide zu starker Korrosion führen. Ein gemeinsames Kennzeichen dieser bekannten Methoden ist der Einsatz von Maleinsäure- und Bernsteinsäureanhydrid, von Malein-, Bernstein- und Fumarsäure sowie von Edelmetall-Katalysatoren. Während der Hydrierung spalten die Anhydride Wasser ab, das noch vorhandenes Anhydrid hydrolysiert und die sehr korrosiv wirkenden Dicarbonsäuren bildet. In der DE-OS 25 53 761 (= US-PS 4 096 156) werden z. B. Ag oder Au und Ru, Rh, Pd, Os, Jr und/oder Pt und/oder ihre Verbindungen als katalytische aktive Substanzen offenbart. Die Hydrogenolyse findet vorzugsweise unter einem Druck von 100 bis 350 bar statt. In der DE-OS 26 02 894 (= GB-PS 1 534 136) wird die Gasphasen-Hydrierung der Maleinsäure und/oder ihrer Anhydride in Gegenwart eines Cu-/Pd-Katalysators beschrieben, die natürlich hohe Temperaturen, 150 bis 350 °C, zur Verdampfung der Substrate verlangt.

0184055
O.Z. 4030/4061

Unter diesen Bedingungen ist die Korrosivität der Dicarbonsäuren besonders hoch. In der US-PS 3 065 243 werden zwar neben Maleinsäure- und Bernsteinsäureanhydrid Ester der Malein-, Bernstein- oder Fumarsäure eingesetzt, die Hydrierung erfolgt aber ebenfalls in der Gasphase mit der sehr geringen Kontaktbelastung von 0,073 Teilen Ester/Teil Katalysator, nach den Beispielen überwiegend bei hohen Temperaturen von 300 bis 330 °C, die einen Kupferchromit-Katalysator in kurzer Zeit desaktivieren.

Die Aufgabe der Erfindung war es deshalb, ein wirtschaftliches Verfahren zur Herstellung von Butyrolacton zu finden, das auf den Einsatz von Säuren bzw. Anhydriden, die nach Wasserabspaltung während der Hydrierung teilweise hydrolysiert werden können, verzichtet und damit keine Korrosionsprobleme aufwirft und mit einem preiswerten Katalysator, der auch als Festbett angeordnet werden kann, bei hoher Belastung und relativ niedrigen Wasserstoffdrücken und relativ niedrigen Temperaturen durchgeführt werden kann.

Diese Aufgabe wird erfindungsgemäß entsprechend den Angaben der Patentansprüche gelöst.

Bei der Hydrierung von Dicarbonsäureestern, wie Maleinsäure-, Fumarsäure- und Bernsteinsäureestern, unterbleibt die Bildung korrosiv wirkender Säuren. Die erfindungsgemäße Wahl der Reaktionsbedingungen führt überraschenderweise bei der Hydrierung in der Rieselphase und bei einer hohen Kontaktbelastung von etwa 0,2 bis 1 Teilen Ester/Teil Katalysator zu der vorzugs- weisen Bildung von $\gamma$-Butyrolacton in weit über 80 %iger Ausbeute und nur zu einem geringen Anfall von Butandiol-1.4, der je nach Einstellung von Wasserstoff- partialdruck und Hydriertemperatur zwischen 1 und 20 %

0184055
O.Z. 4030/4061

schwanken kann. Normalerweise entsteht bei der Hydrierung unter den erfindungsgemäßen Bedingungen der erwähnte Dicarbonsäureester Butandiol-1.4 in nahezu quantitativer Ausbeute. Es war deswegen nicht vorauszusehen, daß es bei Verwendung von Kupferchromit-katalysatoren - selbst bei gezielt eingestellten Hydrierbedingungen - möglich war, $\gamma$-Butyrolacton zum Hauptprodukt werden zu lassen und die Bildung von Butandiol-1.4 nahezu völlig zurückzudrängen.

Die Hydrierung läßt sich sowohl kontinuierlich wie auch diskontinuierlich bis zu einem 100 %igen Umsatz führen. Zur Herstellung der Dicarbonsäureester aus den Anhydriden oder Säuren dienen vorzugsweise Methanol, Ethanol, Propanol, n- oder i-Butanol. Der Einsatz höherer Alkohole ist möglich, führt aber zu einer hohen, nicht wirtschaftlichen Belastung der Hydrieranlagen. Nach Abschluß der Hydrierung werden die Alkohole abgetrennt und dem Veresterungsverfahren wieder zugeführt. Anhydride werden in großem Maßstab z. B. zur Herstellung von Weichmachern verestert. Die bekannten Veresterungsverfahren lassen einen nahezu 100 %igen Umsatz zu und bieten keine Probleme bei der Durchführung. Der erhaltene Rohester kann anschließend direkt in der Rieselphase hydriert werden.

Als Hydrierkatalysatoren können übliche, im Handel befindliche Kupferchromit-Kontakte eingesetzt werden, die z. B. 30 bis 50 Gew.-% CuO, 30 bis 60 Gew.-% $Cr_2O_3$ und 0 bis 10 Gew.-% BaO enthalten können. Bevorzugt setzt man Barium-enthaltende Kupferchromit-Kontakte ein. Die Hydriertemperaturen liegen zwischen 180 bis 260 °C, vorzugsweise bei 200 bis 240 °C. Wasserstoffdrücke von 1 bis 12 bar, vorzugsweise von 2 bis 10 bar, insbesondere von 3 bis 8 bar reichen aus, um einen mehr als 97 %igen Umsatz zu erreichen.

Der Einsatz von Festbettreaktoren, die mit stückigen oder zu Tabletten verformten Katalysatoren beschickt werden, liefert wirtschaftliche Vorteile gegenüber Rührreaktoren, die im allgemeinen nur diskontinuierlich betrieben werden können. Die Hydrierung kann aber auch in Gegenwart pulverförmiger Kupferchromit-Katalysatoren in Autoklaven durchgeführt werden, wenn besondere Umstände das erfordern.

Das erfindungsgemäße Verfahren hat den Vorteil, daß die Einsatzstoffe leicht zugänglich sind und bei der Umsetzung mit Wasserstoff nicht zu korrosiven Medien führen, wie beispielsweise Maleinsäureanhydrid.

Überraschenderweise erhält man die hohen Ausbeuten nur unter den erfindungsgemäßen Bedingungen. Bei höherem Druck, beispielsweise 300 bar, erhält man nur 0,4 % $\gamma$-Butyrolacton neben 95,7 % Butandiol-1.4 (s. Vergleichsbeispiel A). Geringere Ausbeuten und Korrosion erhält man bei der Hydrierung von geschmolzenem Maleinsäureanhydrid, wobei der Katalysator und die Apparatur angegriffen werden (s. Vergleichsbeispiel B).

Beispiel 1

Ein Hochdruckdurchflußreaktor (Länge 120 cm, ∅ 24 mm, konzentrisches Thermorohr ∅ 10 mm) von 400 ml Katalysatorfüllvolumen wird mit 80 ml/h Maleinsäurediisobutylester bei 230 °C und 8 bar Gesamtdruck beschickt. Als Katalysator wird ein handelsüblicher CuCr-Kontakt (z.B. CuO 42 %, $Cr_2O_3$ 40 %, BaO 8 %) eingesetzt. Die Hydrierung erfolgt in der Rieselphase. Der isobutanolfreie Hydrieraustrag enthält neben 0,5 % Vorläufen, 3,5 % n-Butanol, 84 % $\gamma$-Butyrolacton, 10 % Butandiol-1.4, 0,3 % Dibutandiolether und 1 % Ester. Der Rest sind Zwischenläufe.

Beispiel 2

Ausführung wie Beispiel 1, jedoch wird die Hydriertemperatur auf 250 °C erhöht. Der isobutanolfreie Hydrieraustrag enthält

    0,9 % Vorläufe
    8,8 % n-Butanol
   78,3 % $\gamma$-Butyrolacton
    9,4 % Butandiol-1.4
    0,4 % Dibutandiolether
    1,2 % Zwischenläufe
   <0,1 % Ester.

Beispiel 3

Ausführung wie Beispiel 1, jedoch wird die Temperatur auf 210 °C erniedrigt. Der isobutanolfreie Hydrieraustrag setzt sich wie folgt zusammen:

    0,7 % Vorläufe
    3,4 % n-Butanol
   71,8 % $\gamma$-Butyrolacton
    7,2 % Butandiol-1.4
    0,3 % Dibutandiolether
    0,9 % Zwischenläufe
   16,7 % Ester

Beispiel 4

Ausführung wie Beispiel 1, jedoch wird der Druck auf 3 bar gesenkt. Der isobutanolfreie Hydrieraustrag besteht aus:

    0,7 % Vorläufe
    5,8 % n-Butanol

85,2 % $\gamma$-Butyrolacton

1,1 % Butandiol-1.4

0,4 % Dibutandiolether

6,1 % Ester

0,7 % Zwischenläufe


## Beispiel 5

Ausführung wie Beispiel 1, jedoch wird der Druck auf 12 bar erhöht. Hierbei stellt sich folgender isobutanolfreie Austrag ein:

1,0 % Vorläufe

3,9 % n-Butanol

83,4 % $\gamma$-Butyrolacton

9,3 % Butandiol-1.4

0,4 % Dibutandiolether

1,1 % Ester

0,9 % Zwischenläufe


## Vergleichsbeispiel A

Ausführung wie Beispiel 1, jedoch wird der Druck auf 300 bar erhöht. Der isobutanolfreie Hydrieraustrag besteht aus:

0,8 % Vorläufe

1,6 % n-Butanol

0,4 % $\gamma$-Butyrolacton

95,7 % Butandiol-1.4

0,2 % Ester

1,3 % Zwischenläufe

## Beispiel 6

Ausführung wie Beispiel 1, jedoch wird anstelle des Maleinsäurediisobutylesters Fumarsäurediisobutylester eingesetzt. Der erhaltene Hydrieraustrag setzt sich wie folgt zusammen:

0,6 % Vorläufe
4,2 % n-Butanol
82,3 % $\gamma$-Butyrolacton
11,4 % Butandiol-1.4
0,3 % Dibutandiolether
1,2 % Ester

## Beispiel 7

Ausführung wie Beispiel 1, jedoch wird anstelle des Maleinsäurediisobutylesters Bernsteinsäurediisobutylester eingesetzt. Der Hydrieraustrag hat folgende Zusammensetzung:

0,4 % Vorläufe
3,8 % n-Butanol
84,6 % $\gamma$-Butyrolacton
9,9 % Butandiol-1.4
0,4 % Dibutandiolether
0,9 % Ester

Setzt man in den Beispielen anstelle des Diisobutylesters die entsprechenden Dimethyl-, Diethyl-, Di-n-propyl-, Diisopropyl- und Di-n-butylester sowie deren Gemische ein, werden vergleichbare Ergebnisse erhalten.

**0184055**

O.Z. 4030/4061

## Vergleichsbeispiel B

Über den in Beispiel 1 erwähnten Katalysator wird in der gleichen Apparatur bei 210 °C und 8 bar Gesamtdruck 30 ml/h geschmolzenes Maleinsäureanhydrid geleitet. Der Hydrieraustrag ist von grüner Farbe, hervorgerufen durch herausgelöste Katalysatorbestandteile (110 ppm $Cr_2O_3$, 21 ppm CuO; 7 ppm $Fe_2O_3$ - stammt aus der Apparatur). Er setzt sich wie folgt zusammen:

$\quad$ 11,9 % $H_2O$
$\quad$ 0,2 % Vorläufe
$\quad$ 8,4 % Tetrahydrofuran
$\quad$ 47,9 % $\gamma$-Butyrolacton
$\quad$ 7,2 % Butandiol-1.4
$\quad$ 10,3 % Bernsteinsäureanhydrid
$\quad$ 2,7 % Ester
$\quad$ 1,8 % Butoxybernsteinsäuredi-n-butylester
$\quad$ 2,1 % Zwischenläufe
$\quad$ 7,5 % Bernsteinsäure

Patentansprüche:

1. Verfahren zur Herstellung von $\gamma$-Butyrolacton durch katalytische Hydrierung von Maleinsäure-, Fumarsäure- oder Bernsteinsäureester bzw. deren Gemische, dadurch gekennzeichnet, daß man die Ester in Gegenwart von Kupferchromit-Katalysatoren in der Rieselphase bei einem Wasserstoffdruck von 1 bis 12 bar und Temperaturen von 180 bis 260 °C hydriert.

2. Verfahren nach Anspruch 1, dadurch gekennzeichnet, daß man in Gegenwart eines Barium-enthaltenden Kupferchromit-Katalysators hydriert.

3. Verfahren nach Anspruch 1 und 2, dadurch gekennzeichnet, daß man bei einem Wasserstoffdruck von 2 bis 10 bar, vorzugsweise 3 bis 8 bar, hydriert.

4. Verfahren nach den Ansprüchen 1 bis 3, dadurch gekennzeichnet, daß man bei Temperaturen von 200 bis 240 °C hydriert.

**Europäisches Patentamt**

# EUROPÄISCHER RECHERCHENBERICHT

| | EINSCHLÄGIGE DOKUMENTE | | EP 85114559.9 |
|---|---|---|---|
| **Kategorie** | **Kennzeichnung des Dokuments mit Angabe, soweit erforderlich, der maßgeblichen Teile** | **Betrifft Anspruch** | **KLASSIFIKATION DER ANMELDUNG (Int. Cl 4)** |
| D,X | US - A - 3 065 243 (DUNLOP et al.) <br> * Ansprüche 1,5 * <br> -- | 1,3,4 | C 07 D 307/32/ <br> B 01 J 23/86 |
| A | DE - A1 - 2 501 310 (DEUTSCHE TEXA- CO) <br> * Anspruch 1 * <br> -- | 1 | |
| A | CHEMICAL ABSTRACTS, Band 100, Nr. 21, 21. Mai 1984, Columbus, Ohio, USA <br><br> U. MATTEOLI et al. "Homogeneous catalytic hydrogenation of dicarboxylic acid esters" <br> Seite 581, Spalte 1, Zusammenfassung-Nr. 174 250e <br><br> & J.Mol.Catal. 1984 22(3) 253-62 <br><br> ---- | | |

**RECHERCHIERTE SACHGEBIETE (Int. Cl.4)**

C 07 D 307/00

Der vorliegende Recherchenbericht wurde für alle Patentansprüche erstellt.

| Recherchenort | Abschlußdatum der Recherche | Prüfer |
|---|---|---|
| WIEN | 26-02-1986 | LUX |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82